(19) **Europäisches Patentamt / European Patent Office / Office européen des brevets**

(11) **EP 4 116 989 A1**

(12) # EUROPEAN PATENT APPLICATION

(43) Date of publication:
**11.01.2023 Bulletin 2023/02**

(21) Application number: **22160363.2**

(22) Date of filing: **06.03.2022**

(51) International Patent Classification (IPC):
**G16H 20/30** (2018.01)    **A63B 24/00** (2006.01)

(52) Cooperative Patent Classification (CPC):
**G16H 20/30; A61B 5/0004; A61B 5/1114;**
**A61B 5/6806; G06K 9/00496; G06V 40/23;**
A63B 2220/17; A63B 2220/34; A63B 2220/40;
A63B 2220/56

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.07.2021 PL 43839821**

(71) Applicant: **SHAPE.CARE Sp. z o.o.**
**30-394 Krakow (PL)**

(72) Inventors:
• **KRZYSZKOWSKI, Tomasz**
**31-207 Krakow (PL)**

• **HOLUBOWICZ, Piotr**
**31-348 Krakow (PL)**
• **ZUZIAK, Axel**
**31-352 Krakow (PL)**
• **SIWEK, Michal**
**31-834 Krakow (PL)**
• **KUCHARZ, Piotr**
**30-410 Krakow (PL)**
• **CHWALEK, Kamil**
**31-236 Krakow (PL)**

(74) Representative: **Kancelaria Eupatent.pl Sp. z.o.o**
**Ul. Kilinskiego 185**
**90-348 Lodz (PL)**

(54) **A METHOD AND A SYSTEM FOR MONITORING OF EXERCISES PERFORMED IN GLOVES**

(57)    A method for monitoring of exercises performed in gloves, wherein the method comprises providing a user with a pair of gloves, including a left glove (110) and a right glove (120), and monitoring the performance of the exercise by a data processing device (140). The gloves (110, 120) comprise a measurement module (130), the measurement module (130) comprising: a three-axis accelerometer (133); a three-axis gyroscope (134); at least four pressing force sensors (132) arranged at predetermined positions, wherein the positions of the sensors on the left glove (110) are symmetrical with respect to the positions of the sensors on the right glove (120); and a wireless data transmission module (131). The method comprises performing the following steps, in real time, when the user performs the exercise while wearing the gloves (110, 120): reading (201) the data from the sensors (132, 133, 134) in the gloves (110, 120); transmitting (202) the data read from the sensors (132, 133, 134) to the data processing device (140); processing (203) the transmitted data to analyze differences in data from the sensors of the left glove (110) with respect to the data from the sensors of the right glove (120); and by means of the signaling system (143), providing a feedback to the user, wherein the feedback corresponds to the detected differences (204).

Fig. 1

EP 4 116 989 A1

**Description**

TECHNICAL FIELD

The present invention relates to monitoring and analyzing of exercises performed in gloves, in particular strength workout exercises, such as weightlifting.

BACKGROUND

Weightlifting is a popular form of both professional and recreational workout. Exercisers can track their training progress by recording various parameters, such as the number of repetitions or lifted weight, in order to improve their results. In addition, in order to prevent injuries and to optimize the added benefits of training, the exercisers can monitor exercise performance to ensure proper technique or to select appropriate exercise regimens. However, since it can be difficult for the exercisers to self-assess their own performance in weightlifting without external reference or evaluation, immediate feedback during training would improve the effectiveness of training regimens. Observation of the training is usually carried out by a personal trainer who may instruct the exerciser with suggestions on how to properly perform the exercise. However, such solution is available only for a small group of exercisers, due to the time and costs associated with the involvement of a personal trainer.

There are various types of automated training monitoring systems that use various types of electronic devices.

A US patent application US2017216665A1 describes a system that uses sensors mounted on exercise equipment. However, such system requires use of exercise equipment that must be adapted in a predetermined manner.

A US patent application US20150196803A1 describes a video monitoring system that processes an image of the exerciser and analyzes the exercise on a regular basis, providing appropriate instructions. However, such system requires lots of computing power to process the data.

A US patent application US2018193699A1 describes special gloves with sensors to monitor the number of movements performed by a manual labor worker, but the measurement precision and method of use is not suitable for accurate monitoring of performance of physical exercises.

SUMMARY OF THE INVENTION

There is a need to provide a method of monitoring of exercises, to constantly inform the user about the quality of performance of the exercises in real time, while the preforming the exercises.

The present invention relates to a method for monitoring of exercises performed in gloves. A user is provided with a pair of gloves, including a left glove and a right glove. Each glove (110, 120) comprises a measurement module, the measurement module comprising: a three-axis accelerometer; a three-axis gyroscope; at least four pressing force sensors arranged at predetermined positions, wherein the positions of the sensors on the left glove are symmetrical with respect to the positions of the sensors on the right glove; and a wireless data transmission module. A data processing device is provided, the data processing device comprising: a wireless data transmission module communicatively coupled to the wireless data transmission modules of the gloves; a data processing system; and a signaling system. The method comprises performing the following steps, in real time, when the user performs the exercise while wearing the gloves: reading the data from the sensors in the gloves; transmitting the data read from the sensors to the data processing device; processing the transmitted data to analyze differences in data from the sensors of the left glove with respect to the data from the sensors of the right glove; and by means of the signaling system, providing a feedback to the user, wherein the feedback corresponds to the detected differences.

The presented method allows to check whether a user performs the exercises correctly. In particular, by measuring the distribution of forces acting on gloves (i.e., force measured by individual pressing force sensors), it can be checked whether the user correctly grips (i.e., places the hand on the grip) and lifts weights. Moreover, by measuring the positioning plane (using an accelerometer) of the hand, it allows to determine anomalies and errors in work of hands while exercising. By comparing the data from the sensors of the left and right glove, it allows to determine the balance of forces between the left hand and the right hand. As a result, when presenting the data to the user, the user can be informed, for example, to correct the position of one hand in relation to the other hand for better exercise results, in particular so as to distribute the weight more evenly being lifted between the right and left hand.

By analyzing the values of signals from the sensors over time, the method can be used to recognize individual repetition cycles of exercise, i.e., the beginning and end of the repetition cycle. As a result, it is possible to determine the duration of the repetition, the number of repetitions in a set and the time interval between the individual repetitions. The results of the analysis of the change in duration of each repetition over several sets are presented to the user. This information makes it possible to evaluate the dynamics of the performed sets and by comparing it with the previous set or typical values for a given exercise stored in the device's memory, it is possible to assess the quality of the performed exercises in relation to previously performed exercises.

In particular, the length of the intervals between the repetitions and between the sets allows to determine the degree of the tiredness of the user.

The three-axis accelerometer detects the change in the position of the hand during the exercise, and the pressing force sensors detect the pressing force corresponding to the lifted load on individual locations on the hand.

Thanks to the use of a three-axis gyroscope, it is possible to detect the angular position of the user's hand while exercising.

The wireless interface can be an interface compatible with one of the common data transmission standards, for example Bluetooth, Wi-Fi, or an own transmission system dedicated to a given system.

The data processing device may be a smartphone that operates an application handling the functions of the data processing system. The smartphone provides a communication interface (for example, Bluetooth) and computing power to process sensor data, and allows the measurement data and the results of the analysis of the measurement data to be stored to create an exercise and training history. In this way, the functionality of the system can be made available to a wide range of smartphone users.

The data processing device may be also a tablet or a TV set executing an application that performs the functions of a data processing system. The tablet or the TV set may constitute an equipment assigned to the exercise performance location (for example, in a gym, each exercise station may have a dedicated tablet or TV installed) to enable the use of the system by users who do not have their own smartphone with the application or to provide feedback to users via a relatively large screen.

It is also possible to provide dedicated terminals to support the functionality of the data processing device.

The signaling system may comprise a display. By means of the display, the user can be informed about the current course of the exercises, suggestions for improving the performance of these exercises, etc. The information can be displayed by means of text or graphics, for example in the form of graphs.

The signaling system may comprise a sound emitter. By means of the sound emitter, the user can get a feedback in the form of voice commands or tone signals. Then the user does not need to observe the display. The sound emitter can be a loudspeaker that emits sound to the environment, or it can be in the form of earphones or headphones.

The signaling system may include a vibrating element built into the glove. By means of the vibrating system, the user can get a feedback, suggesting, for example, to increase the intensity of the work of the weaker hand (for example, by vibrating the element in the particular glove at a certain rhythm).

Alternatively, it is possible to use a vibrating element which is already present on the smartphone to provide feedback to the user. For example, the smartphone may be in the exerciser's pocket, and the information on the right hand is signaled with a different rhythm than the information on the left hand.

The glove may comprise a pulse oximeter to measure the pulse rate and blood oxygen saturation.

Each glove may contain four pressing force sensors located respectively on the middle finger near the middle phalanx bone, on the forefinger near the proximal phalanx, near the second metacarpal bone and near the fifth metacarpal bone. The present inventors have found that such placing of the sensors allows for optimal monitoring of most of the popular weight exercises discussed in the embodiments.

The accelerometer may be adapted to detect a finger tap on a glove. This allows the user to intuitively control the system by issuing appropriate commands corresponding to the type of tap, for example a weaker or stronger tap, single or multiple, short, or long-held taps. These commands can activate various system functions, such as starting and stopping a measurement (to optimize battery life), displaying historical exercise results, etc.

At least one glove may comprise with a pedometer so that it is possible to determine the number of steps taken by the user. In addition, by providing parameters that allow to determine the length of the user's step, it is possible to calculate the length of the route that the user has made.

The measurement module may include a computing module for preprocessing the sensor data. Owing to this, the algorithms perform the initial data processing and draw conclusions from the measurements, while wearing the glove, in the measurement module, and the application (for example, an application on a smartphone) draws wider and more accurate conclusions on their basis by analyzing data from many devices and data from previous sets.

The invention also corresponds to a system for monitoring of exercises performed in gloves, comprising a pair of gloves and a data processing device (140) having a structure and configured to operate as described above.

These and other features, aspects and advantages of the invention will become better understood with reference to the following drawings, descriptions, and claims.

BRIEF DESCRIPTION OF DRAWINGS

The present invention is shown by means of preferable embodiments in a drawing, wherein:

Fig. 1 shows a block diagram of an exercise performance monitoring system;
Fig. 2 shows the steps of a method for exercise performance monitoring;
Fig. 3A shows a glove with a sensor system;
Fig. 3B shows the arrangement of the measurement points on the inner side of the hand;
Fig. 4 shows the sensor system in cross-section;
Fig. 5 is an exploded view of the sensor system;
Fig. 6A shows the flexible mount for the sensor system;

Fig. 6B shows a flexible strip with conductive tracks;

Fig. 6C shows a 'pogo' type connector;

Fig. 7A shows schematic top view of a fabric glove with conductive threads;

Fig. 7B shows schematically of a woven glove with an exemplary arrangement of pressing force sensors on the inner side of the glove;

Fig. 8 shows an example reading of data from accelerometers during an exercise of lifting a dumbbell sideways while standing;

Fig. 9 shows an example of reading data from a single pressing force sensor on the left and right gloves during an exercise involving lifting dumbbells sideways while standing;

Fig. 10 shows an example reading of data from accelerometers during a deadlift exercise;

Fig. 11 shows an exemplary reading of data from a single pressing force sensor on the left and right gloves during a deadlift exercise;

Fig. 12 shows an example reading of accelerometer data during a dumbbell lifting exercise for a biceps exercise;

Fig. 13 shows an exemplary reading of data from a single pressing force sensor on the left and right gloves during a dumbbell lifting exercise for a biceps exercise;

Fig. 14 shows the distribution of forces acting on the hand when performing certain types of exercises to indicate which sensors play the most important role in monitoring the particular exercise.

DETAILED DESCRIPTION

The description presented herein is not to be taken in a limiting sense but is made merely for the purpose of illustrating the general principles of the invention.

The embodiment presented herein contains all features foreseen by the present disclosure. However, other embodiments are feasible as well that do not contain all features necessary to achieve particular technical advantages corelated with such feature.

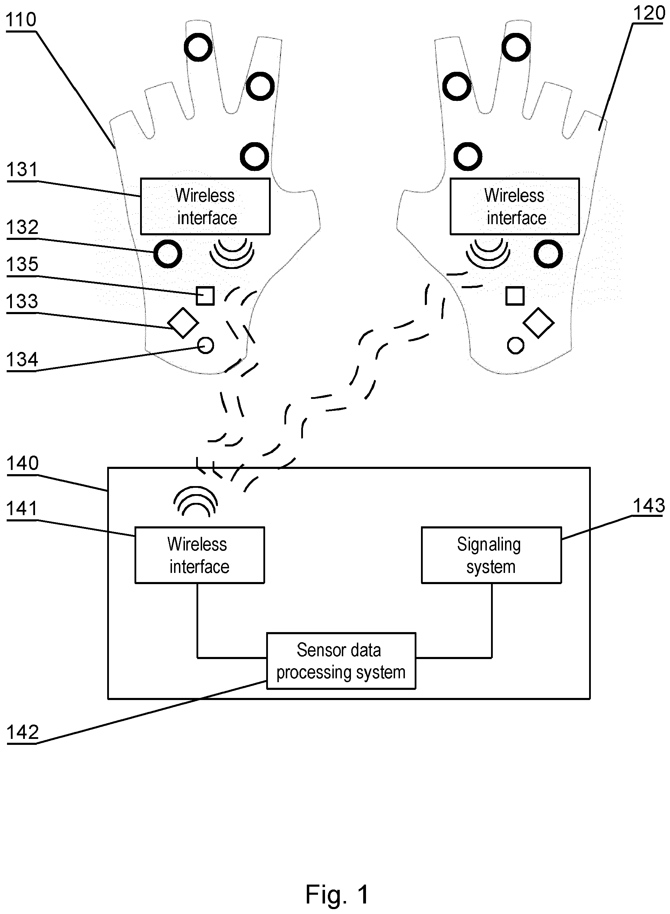

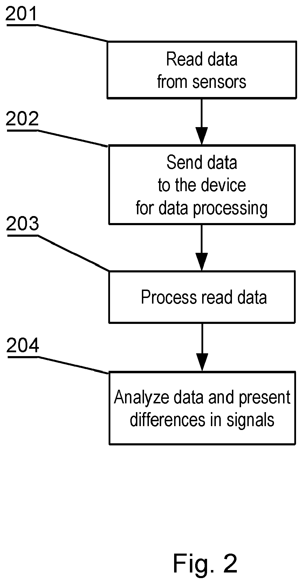

Fig. 1 shows a block diagram of an exemplary exercise performance monitoring system with which the exercise performance monitoring method shown in Fig. 2 may be implemented.

The user performs the exercises with a pair of gloves: a left glove 110 and a right glove 120. Each glove comprises a measurement module 130 which includes a three-axis accelerometer 133, a three-axis gyroscope 134, at least four pressing force sensors 132 arranged at certain positions symmetrically on the left glove 110 with respect to the right glove 120 and wireless data transmission module 131. The gloves communicate with data processing device 140 which includes wireless data transmission module 141 connected to wireless data transmission modules 131 in gloves 110, 120, data processing circuit 142 and signaling system 143.

During performance of the exercises by the user wearing gloves 110, 120, the method comprises the steps of reading (step 201) data from the sensors 132, 133, 134 in real time in the data processing device 140, transmitting (step 202) the data read from sensors 132, 133, 134 to data processing device 140, and processing (step 203) the transmitted data to analyze differences in the data from the sensors in the left glove 110 with respect to the data from the sensors in the right glove 120. By means of the signaling circuit 143 the feedback is communicated (step 204) to the user depending on the detected differences.

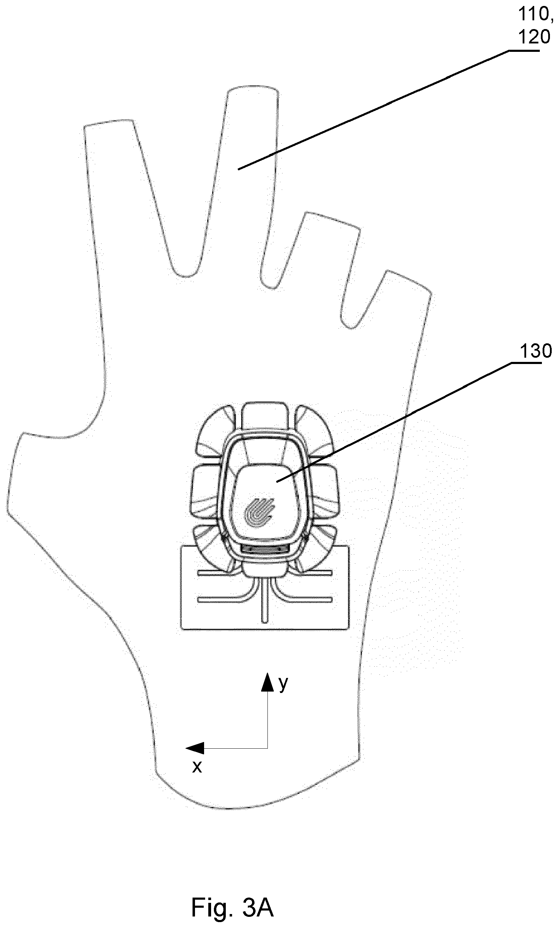

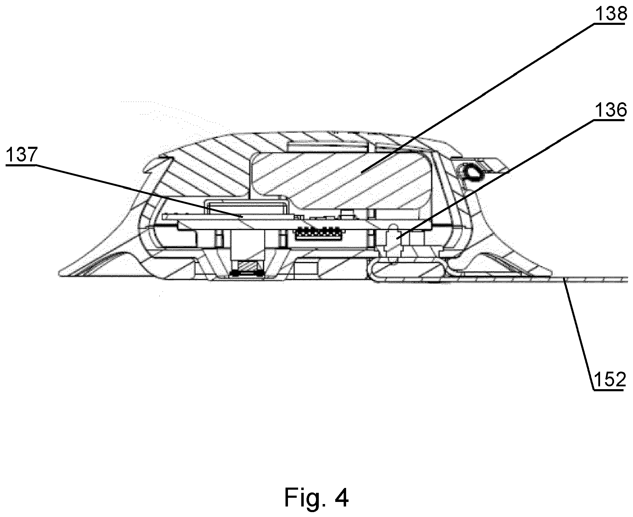

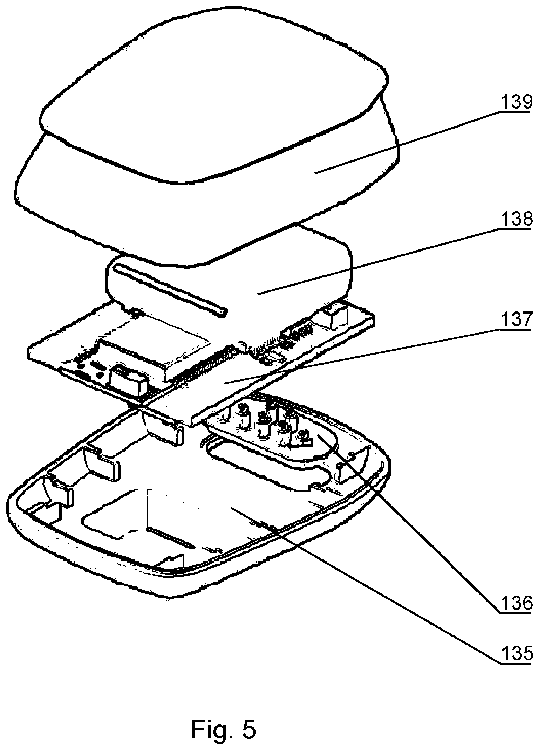

The measurement module 130 constituting the sensor system may have a form of a PCB (printed circuit board) 137 to which by means of flexible conductive threads and by means of the connectors 136 pressing force sensors 132 are connected. Preferably, an accelerometer 131 is provided on a printed circuit board 137. The measurement module 130 is powered by a battery 138, which, along with the circuit board 137, is located in a housing that includes a lower portion 135 and an upper portion 139 as shown in Figs. 3A, 4, and 5.

The design of the housing protects the measurement module 130 against accidental crushing (e.g., when a glove with the module lying on the ground is stepped on) or hitting a hard surface.

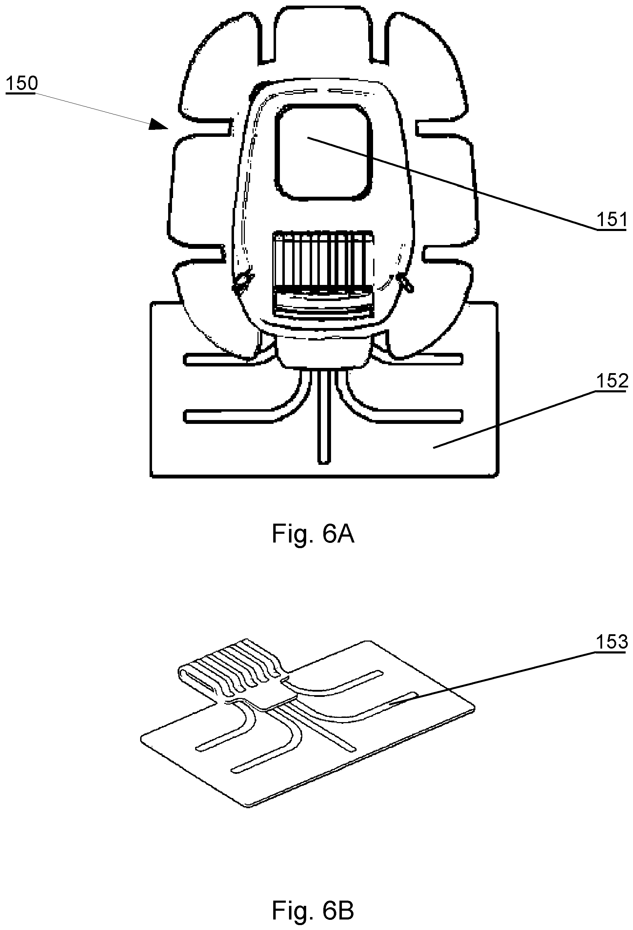

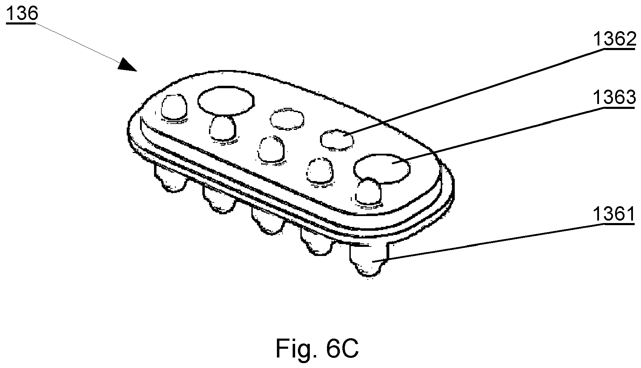

As shown in Figs. 6A-6C, the connector 136 preferably is in the form of a 'pogo' type connector in which resilient communication pins 1361 abut a flexible band 152 with conductive tracks 153 to which conductive threads from sensors are connected. In addition, the connector 136 has two charging pins 1362 of the measurement module 130, and magnets 1363 for attaching the charger plug. The glove has a pulse oximeter attached to the circuit board 137 such that its measurement beam passes towards the user's skin through the pulse oximeter opening 151 located in the flexible mount 150.

Preferably, the measurement module 130 is located in a flexible mount 150, which in turn is attached to the glove 110, 120 e.g., by glue or is sewn into the material of the glove (or placed in a special pocket in the glove material). As a result, the measuring module 130 can be easily separated from the glove 110, 120, e.g., for the purpose of washing gloves.

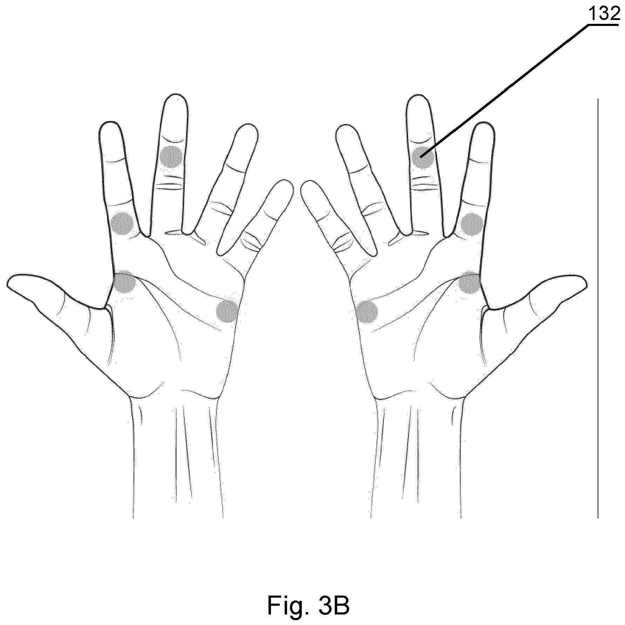

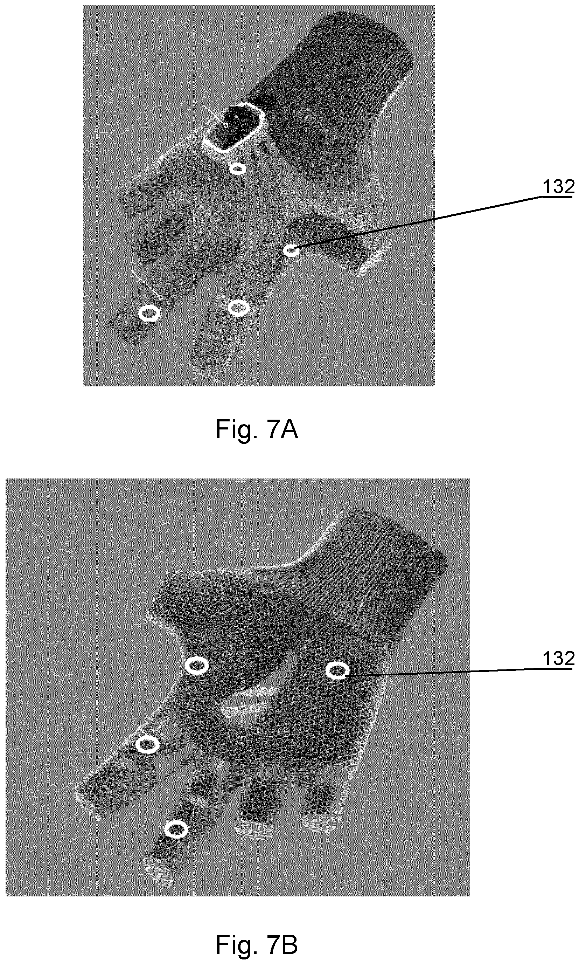

An exemplary arrangement of pressing force sensors 132 on the inside of the glove is shown in Figs. 7A and 7B. Fig. 3B shows the arrangement of the measurement points on the inner side of the hand.

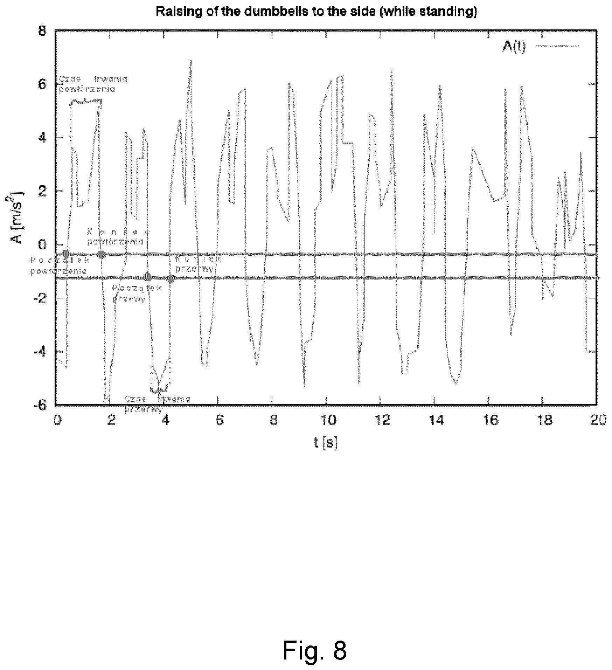

Fig. 8 shows an example reading of data from accelerometers during an exercise of lifting a dumbbell sideways while standing.

Based on the above graph, the algorithm determines the number of repetitions, the duration of each repetition and the length of the intervals between repetitions.

After starting the set, by using the data from the accelerometer for each time step, the device calculates the resultant length of the acceleration vector according to the following formula:

$$A_w = \sqrt{A_x^2 + A_y^2 + A_z^2}$$

The $A_w$ values are stored in the device memory (e.g., on a smartphone). The string of $A_w$ values is averaged using the following moving average formula:

$$A_{sr} = \frac{A_{w0} + A_{w1} + \cdots + A_{wn}}{n},$$

where n is the number of periods determined empirically in order to minimize the measurement errors of the accelerometer.

The algorithm monitors the $A_{sr}$ values and if its absolute value exceeds the established $A_{pr}$ threshold (determined empirically), it will mark the given measuring point as the start of the repetition. All subsequent $A_{sr}$ values greater than the $A_{pr}$ threshold are taken as values during the repetition duration. Likewise, the point at which $A_{sr}$ falls below the $A_{pr}$ threshold is considered the end of the repetition.

In general, the data readings from the left glove and right glove accelerometers are substantially similar, so that only a single graph is shown in Fig. 8. In addition, the graph (read values from accelerometers) allows to determine the dynamics of the exercises/repetitions. For example, the falling dynamics of repetitions may be a sign of increasing user tiredness.

In addition, by comparing the readings of a given set with the previous sets or the typical values for a given exercise, it can be determined whether the user is improving or deteriorating in the exercises. As a result, for example, it can generate a message for the user: "You performed much worse in this set, you are already tired."

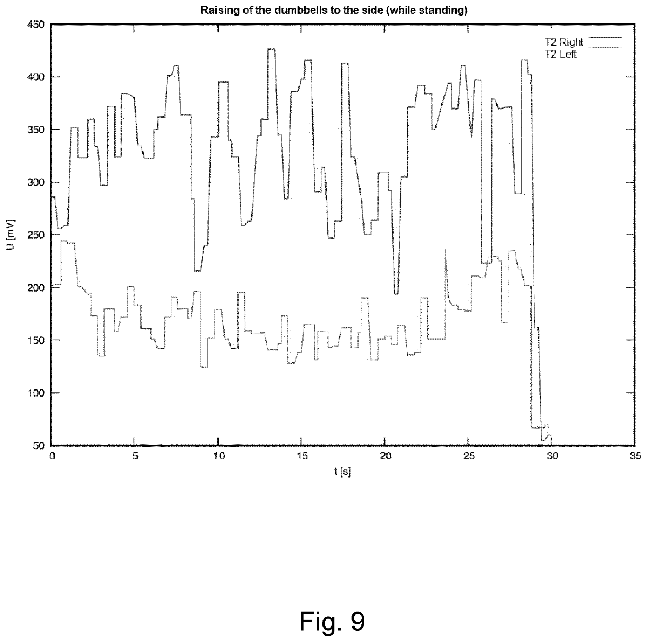

Fig. 9 shows an exemplary reading of data from a single pressing force sensor on the left and right gloves during an exercise involving lifting a dumbbell sideways while standing.

By comparing the plots from the pressing force sensor (strain gauge) data, it is possible to calculate the "total force" applied to the weight by summing (integrating) the area under the plots from each pressing force sensor 132 and comparing the left and right glove. In the above graph, the area under the graph from the right glove is larger than the area under the graph from the left glove, which means that the share of the right hand and at the same time the balance for the right hand is greater.

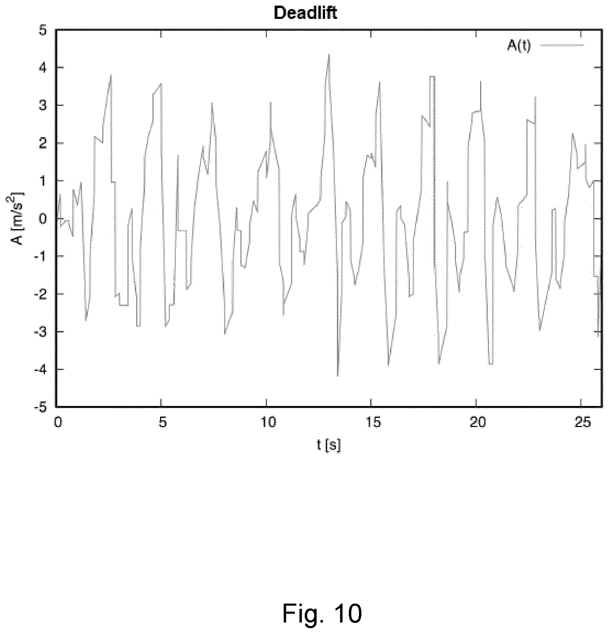

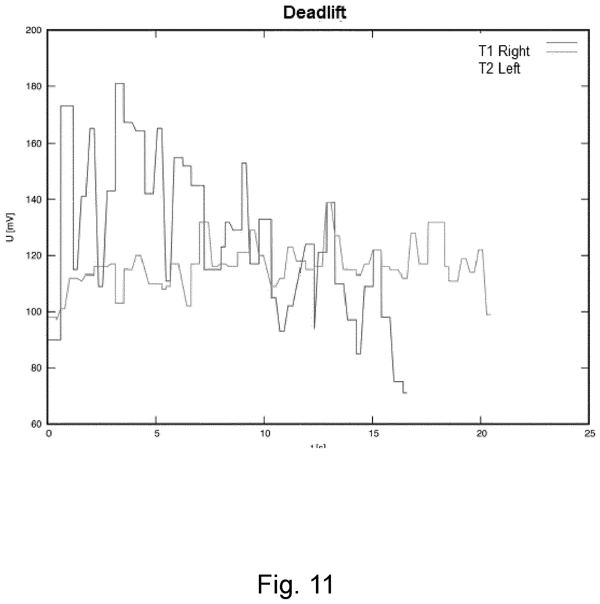

Analogously to Figs. 8 and 9, Fig. 10 respectively shows an exemplary reading of data from the accelerometers during a deadlift exercise. Fig. 11 shows an exemplary reading of data from a single pressing force sensor on the left and right gloves during a deadlift exercise. From the read data, the system algorithm counts the number of repetitive cycles as the number of repetitions along with the duration of each repetition. The results of the analysis of the change in duration of each repetition over several sets are presented to the user. The pressing force values from the pressing force sensors for the left and right hands are quite even in this case. It can be seen a very even balance between the left and right hands, which is below 5% (in particular, in the middle of the graph).

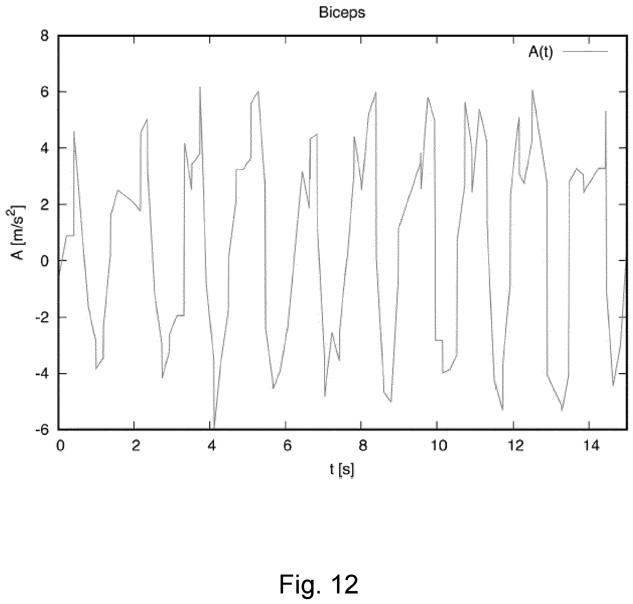

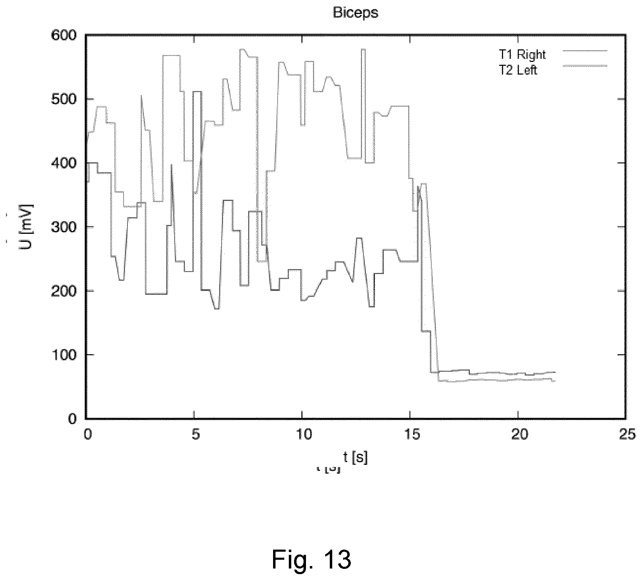

Analogous to Figs. 8-11, Fig. 12 shows an exemplary reading of the accelerometer data during a dumbbell lifting exercise for a ten repetitions biceps exercise. Fig. 13 shows an exemplary reading of data from a single pressing force sensor on the left and right gloves during a dumbbell lifting exercise for a biceps exercise, from which a strong dominance of the left hand can be observed.

After each set of exercises, the system presents a summary message with information on the amount of performed exercises, heart rate value during the exercise, including average heart rate and the heart rate zone in which the exercises were performed (heart rate zone - percentage of maximum heart rate), balance percentage of left to right hand.

In addition, after finishing the training, full statistics on the course of training are displayed (number of exercises, repetitions, sets), the level of fatigue, information about the dominant hand, the quantitative assessment of the training. For example, the quality of the entire training can be expressed as a percentage, compared to previously collected statistics.

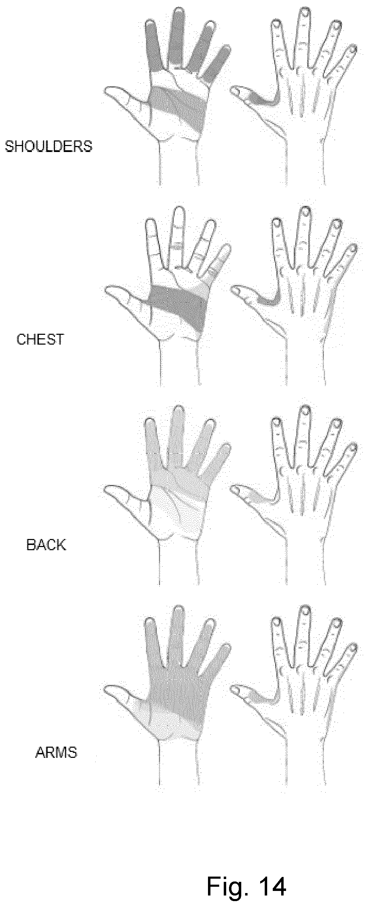

Figure 14 shows the distribution of forces acting on the hand when performing certain types of exercise to indicate which sensors play the most important role in monitoring the exercise. As a result, it is possible to present a hand heat map that is related to the pressing force distribution.

In particular, during exercise, the system presents information on the number of repetitions, the current heart rate and pulse zone, blood saturation, as well as it can graphically present four points on the hand, corresponding to four pressing force sensors on the gloves, which visually show the pressing force value and a light signal (green, yellow, red) symbolizing the current deviations and irregularities of the handle.

While the invention has been described with respect to an embodiment, it will be appreciated that many variations, modifications, and other applications of that embodiment may be made. Therefore, the claimed invention as recited in the claims that follow is not limited to the embodiment described herein.

**Claims**

1. A method for monitoring of exercises performed in gloves, wherein the method comprises providing a user with a pair of gloves, including a left glove (110) and a right glove (120), and monitoring the performance of the exercise, **characterized by**:

- providing the gloves (110, 120) that comprise a measurement module (130), the measurement module (130) comprising:

  - a three-axis accelerometer (133);
  - a three-axis gyroscope (134);
  - at least four pressing force sensors (132) arranged at predetermined positions, wherein the positions of the sensors on the left glove (110) are symmetrical with respect to the positions of the sensors on the right glove (120); and
  - a wireless data transmission module (131);

- providing a data processing device (140), the data processing device (140) comprising:

  - a wireless data transmission module (141) communicatively coupled to the wireless data transmission modules (131) of the gloves (110, 120);
  - a data processing system (142); and
  - a signaling system (143);

- performing the following steps, in real time, when the user performs the exercise while wearing the gloves (110, 120):

  - reading (201) the data from the sensors (132, 133, 134) in the gloves (110, 120);
  - transmitting (202) the data read from the sensors (132, 133, 134) to the data processing device (140);
  - processing (203) the transmitted data to analyze differences in data from the sensors of the left glove (110) with respect to the data from the sensors of the right glove (120); and
  - by means of the signaling system (143), providing a feedback to the user, wherein the feedback corresponds to the detected differences (204).

2. The method according to claim 1, wherein the data processing device (140) is a smartphone operating an application executing the functions of the data processing circuit (142).

3. The method according to claim 1, wherein the data processing device (140) is a tablet or a TV set operating an application performing the functions of the data processing circuit (142).

4. The method according to claim 1 or 2, wherein the signaling system (143) comprises a display.

5. The method according to any of the preceding claims, wherein the signaling circuit (143) comprises an audio emitter.

6. The method according to any of the preceding claims wherein the signaling system (143) comprises a vibrating element embedded within the glove (110, 120).

7. The method according to any of the preceding claims wherein the glove (110, 120) comprises a pulse oximeter configured to measure the pulse and blood oxygen saturation of the user.

8. The method according to any of the preceding claims wherein each glove (110, 120) comprises four pressing force sensors (132) in the following arrangement: a first sensor arranged at the middle finger near the middle phalanx bone, a second sensor arranged at the index finger near the proximal phalanx bone, a third sensor arranged at the second metacarpal bone and a fourth sensor arranged at the fifth metacarpal bone, when the glove is positioned on the hand of the user.

9. The method according to any one of the preceding claims wherein the accelerometer (133) is adapted to detect a finger tap on the glove.

10. The method according to any of the preceding claims wherein at least one glove (110, 120) is provided with a pedometer.

11. The method according to any one of the preceding claims wherein the measurement module (130) comprises a computing module for preprocessing the sensor data.

12. A system for monitoring of exercises performed in gloves, comprising a pair of gloves and a data processing device (140) having a structure and configured to operate according to any of claims 1-11.

EP 4 116 989 A1

110

120

131

Wireless
interface

Wireless
interface

132

135

133

134

140

141

Wireless
interface

Signaling
system

143

Sensor data
processing system

142

Fig. 1

7

201     Read data
from sensors

202     Send data
to the device
for data processing

203     Process read data

204     Analyze data and present
differences in signals

Fig. 2

110,
120

130

y

x

Fig. 3A

132

Fig. 3B

138

136

137

152

Fig. 4

139

138

137

136

135

Fig. 5

Fig. 6A

Fig. 6B

136

1362

1363

1361

Fig. 6C

Fig. 7A

Fig. 7B

Raising of the dumbbells to the side (while standing)

Fig. 8

Raising of the dumbbells to the side (while standing)

Fig. 9

Fig. 10

Fig. 11

Biceps

Fig. 12

Fig. 13

SHOULDERS

CHEST

BACK

ARMS

Fig. 14

Europäisches
Patentamt

European
Patent Office

Office européen
des brevets

# EUROPEAN SEARCH REPORT

Application Number

EP 22 16 0363

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2018/193699 A1 (DEBATES SCOTT P [US] ET AL) 12 July 2018 (2018-07-12) | 1-8, 10-12 | INV. G16H20/30 A63B24/00 |
| Y | * [0008], [0016]-[0017], [0019], [0021]-[0023], [0026], [0028], [0030]-[0031], [0038], [0040]-[0042], [0046]; figures 1-3 * | 9 | |
| X | US 2019/099123 A1 (ZAMBRISKI SHARON ANN [US] ET AL) 4 April 2019 (2019-04-04) | 1-8, 10-12 | |
| Y | * [0005], [0018]-[0021], [0025]-[0026], [0036], [0039]; figures 1-3, 6-7 * * [0006], [0028]; figure 9 * | 1-8, 10-12 | |
| Y | US 2018/333079 A1 (SZÉKELY LAJOS [HU] ET AL) 22 November 2018 (2018-11-22) * [0011], [0037], [0045], [0047]-[0049]; figures 1, 6, 8-9 * | 1-8, 10-12 | |
| Y | CN 111 752 393 A (LI FEIXIANG) 9 October 2020 (2020-10-09) * "Summary of the invention"; figure 5 * | 9 | TECHNICAL FIELDS SEARCHED (IPC) G16H A63B |

The present search report has been drawn up for all claims

| Place of search | Date of completion of the search | Examiner |
|---|---|---|
| Munich | 2 June 2022 | Werner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 0363

This annex lists the patent family members relating to the patent documents cited in the above-mentioned European search report.
The members are as contained in the European Patent Office EDP file on
The European Patent Office is in no way liable for these particulars which are merely given for the purpose of information.

02-06-2022

| Patent document cited in search report | Publication date | Patent family member(s) | Publication date |
|---|---|---|---|
| US 2018193699 A1 | 12-07-2018 | NONE | |
| US 2019099123 A1 | 04-04-2019 | NONE | |
| US 2018333079 A1 | 22-11-2018 | BR 112018009481 A2 | 05-02-2019 |
| | | CA 3005000 A1 | 18-05-2017 |
| | | EP 3401873 A1 | 14-11-2018 |
| | | JP 6837484 B2 | 03-03-2021 |
| | | JP 2019500083 A | 10-01-2019 |
| | | US 2018333079 A1 | 22-11-2018 |
| | | WO 2017081497 A1 | 18-05-2017 |
| | | ZA 201803835 B | 25-09-2019 |
| CN 111752393 A | 09-10-2020 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017216665 A1
- US 20150196803 A1

- US 2018193699 A1